(19)

**Europäisches Patentamt**

**European Patent Office**

**Office européen des brevets**

(11) **EP 4 692 307 A1**

(12) **EUROPEAN PATENT APPLICATION**
published in accordance with Art. 153(4) EPC

(43) Date of publication:
**11.02.2026 Bulletin 2026/07**

(21) Application number: **23937231.1**

(22) Date of filing: **25.10.2023**

(51) International Patent Classification (IPC):
***C12N 1/16*** (2026.01)      ***C12R 1/645*** (2006.01)

(52) Cooperative Patent Classification (CPC):
**A23K 10/16; A23K 10/18; A23L 31/10; C12N 1/16;**
**C12R 2001/645**

(86) International application number:
**PCT/CN2023/126403**

(87) International publication number:
**WO 2024/234556 (21.11.2024 Gazette 2024/47)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **13.05.2023  CN 202310540115**

(71) Applicant: **Angel Yeast Co., Ltd**
**Yichang, Hubei 443003 (CN)**

(72) Inventors:
• **SUN, Yafang**
  **Yichang, Hubei 443003 (CN)**

• **QIN, Xianwu**
  **Yichang, Hubei 443003 (CN)**
• **GUO, Tianfen**
  **Yichang, Hubei 443003 (CN)**
• **ZHANG, Yan**
  **Yichang, Hubei 443003 (CN)**
• **SHI, Yu**
  **Yichang, Hubei 443003 (CN)**

(74) Representative: **karo IP**
**Patentanwälte PartG mbB**
**Steinstraße 16-18**
**40212 Düsseldorf (DE)**

Remarks:
The complete document including Reference
Table(s) and the Sequence Listing(s) can be
downloaded from the EPO website

(54) **KLUYVEROMYCES MARXIANUS AND USE THEREOF**

(57)     The present invention relates to the technical field of fermentation, and provides *Kluyveromyces marxianus* and a use thereof. The accession number of *Kluyveromyces marxianus* is CCTCC No: M2023217. The original strain has good lactic acid resistance and low pH value tolerance. The strain obtained by mutagenesis by using the original strain has organic acid resistance and low pH value tolerance. For example, the original strain can tolerate lactic acid having a concentration of 5.5 wt% and can grow under the condition that the pH value is 2, and particularly, the tolerance of the original strain to lactic acid having a concentration of 4.5 wt% to 5.5 wt% reaches 80%-90%. The original strain can effectively transform lactic acid into bacteria and bacteria proteins, has more efficient lactic acid utilization capacity in corn steep liquor fermentation, can increase the additional value of the corn industry, achieves high-value utilization of low biomass resources, reduces, as an alternative protein, the pressure on environmental resources caused by the traditional breeding industry, meets the protein requirements of global population and the field of feed, and achieves economic development and environmental protection of the fermentation industry.

Fig. 1

EP 4 692 307 A1

**Description**

**CROSS-REFERENCE TO RELATED APPLICATION**

The present application claims the priority of the earlier application document filed at the China National Intellectual Property Office on May 13, 2023, with the application number 202310540115.9, which is incorporated herein by reference in its entirety.

**Technical Field**

The present invention relates to the technical field of fermentation, and more particularly, to *Kluyveromyces marxianus* and a use thereof.

**Background Art**

By 2050, the world's population is expected to reach 9 billion, and the contradiction between food access and population growth will remain prominent, forming a huge "protein gap" between globally available proteins and expected demands in the next 30 years, with an estimated protein gap of 250 million tons by 2050. Proteins are mainly derived from animal proteins and plant proteins, but both the animal proteins and plant proteins need to be bred or planted in certain land areas. However, land resources are facing problems such as land destruction, water and soil erosion, soil salinization and desertification. Therefore, an alternative protein needs to be found to meet the protein requirements of nearly 9 billion people.

At present, alternative proteins have become an important topic in the food industry. The global demand for alternative proteins is surging, and the total scale of the alternative protein market will grow at a double-digit rate every year, and is expected in the market to reach 2.6 billion yuan in 2023. Common alternative proteins mainly include microorganisms and algae. Compared with animal and plant proteins, microbial proteins have the advantages of nutritional comprehensiveness, clean labels, sustainable environmental friendliness, etc. The microorganisms generally used for the production of bacteria proteins include bacteria, fungi and protists, among which a yeast protein is a typical representative, with non-negligible potential ability and development space, and the most popular yeast species are *Saccharomyces cerevisiae, Candida oleophila, Kluyveromyces marxianus,* etc. *Kluyveromyces marxianus* is an unconventional yeast commonly found in dairy products. *Kluyveromyces marxianus* was approved as a new food ingredient in China as early as 2013 and was approved to be added to the "List of Cultures Available in Food" in 2022. This strain has the advantages of broad-spectrum substrate utilization, heat resistance, fast growth rate, special flavor, etc., and can be used in biofuels, brewing, dairy products, condiments, baking, feed and other fields.

In recent years, with the rapid development of animal husbandry in China, feed resources are becoming increasingly scarce, and abundant low-value biomass such as grain processing by-products, waste residues and agricultural and sideline product wastes are discarded and incinerated, resulting in waste of resources. Therefore, achieving high-value utilization of biomass is an inevitable trend in the development of feed. China is a major producer of corn starch. Corn steep liquor, as one of the by-products in the wet production process of corn starch, is rich in sugars, soluble proteins, a variety of amino acids, polypeptides, vitamins and other organic compounds, but is limited in application due to the presence of components such as lactic acid. Therefore, the development of *Kluyveromyces marxianus* that can tolerate and be efficiently transformed into bacteria proteins using lactic acid can improve the additional value of the corn industry, achieve the high-value utilization of low biomass resources, reduce, as an alternative protein, the pressure on environmental resources brought by the traditional aquaculture industry, alleviate the protein requirements of the global population and the field of feed, has better adaptability in food and industrial high-temperature fermentation environments compared with *Saccharomyces cerevisiae,* can reduce the cooling cost, and achieve the economic development and environmental protection of the fermentation industry. It can be seen that *Kluyveromyces marxianus* is a microorganism with a great developing potential.

The Chinese patent application CN115287202A discloses a *Kluyveromyces marxianus* strain and a use thereof in the preparation of functional feed, wherein *Kluyveromyces marxianus* is made into a bacterial solution form. The fermentation microbial agent also includes bacterial solutions of *Bacillus subtilis* and *Bacillus coagulans* and is used for performing solid-state fermentation on a solid fermentation medium containing traditional Chinese medicine residues. The functional feed can effectively promote the animal growth, improve the animal body immunity, reduce the diarrhea rate and mortality rate, improve the feed conversion rate, also regulate the metabolism, promote the growth, and improve the meat quality when used as livestock and poultry feed or a feed additive. It is functional protein feed which is prepared by taking radix bupleuri residues as a main raw material and performing solid-state fermentation in the form of a compound microbial agent. The viable count, pH, odor, moisture, polysaccharides, crude proteins, crude fibers, organic acids and other indexes of each bacterium in the fermentation process are detected, and a feeding test is carried out to investigate the effect on the growth performance of fattening pigs. However, the characteristics of *Kluyveromyces marxianus* are not described in detail, so it is not clear whether *Kluyveromyces marxianus* can improve a fermentation effect and can only be applied in the field of functional feed.

The China patent application CN115211490A provides a new industrial mass production mode of a yeast culture in which the yeast culture is prepared by taking *Kluyveromyces marxianus* as a main bacterium by means of a bacterial and enzyme synergistic fermentation technology. The yeast culture contains aromatic substances such as alcohols and esters and flavoring substances such as nucleotides, polypeptides and free amino acids, has unique flavor and nutritional characteristics, can increase the food consumption of animals and is beneficial to the improvement of production performances, and compared with a *Saccharomyces cerevisiae* culture, solves the problems that after fermentation, wet cooking wine is strong in taste, and animal ingestion is affected. The yeast culture is widely used in animal feed, and can significantly enhance the antioxidant function and immunity of animal bodies, promote the animal growth and improve the animal product quality. However, the crude protein content in this microbial agent is 19.84% after the addition of complex enzymes, and the substrate utilization and protein production ability of a *Kluyveromyces marxianus* strain are not evaluated.

The Chinese patent application CN 107287127A discloses a strain of lactic acid-tolerant ester-producing yeast, which is classified and named *Pichia kudriavzevii,* is capable of tolerating 0-15% lactic acid and metabolizing to generate a plurality of ethyl ester volatile flavor substances, such as ethyl acetate, ethyl propionate and phenethyl acetate in an environment of 0-12% lactic acid, is a brewing functional strain with excellent performances and is only applied in the field of brewing.

The Chinese patent CN 113583883B discloses a strain of ester-producing *Debaryomyces hansenii* which is isolated from Castanea mollissima. This strain has the properties of high temperature resistance, lactic acid resistance, ethanol resistance, high ester production, etc. Specific indexes are as follows: this strain is capable of tolerating a high temperature of 50°C, has lactic acid tolerance of 2%, ethanol tolerance of 8%, and ester production of 1.7 g/L, can be applied to the fermentation of brewed wine, and can effectively improve the flavor and quality of finished brewed wine, but can only be used in the field of winemaking.

**Summary of the Invention**

In view of the above technical problems existing in the prior art, the present application provides *Kluyveromyces marxianus* and an use thereof. This strain can tolerate and be efficiently transformed into bacteria proteins using lactic acid, can improve the additional value of the corn industry, achieve the high-value utilization of low biomass resources, reduce, as an alternative protein, the pressure on environmental resources brought by the traditional aquaculture industry, alleviate the protein requirements of global population and field of feed, and achieve the economic development and environmental protection of the fermentation industry.

Specifically, the present application provides the following technical solutions.

The present application provides *Kluyveromyces marxianus,* the accession number of the *Kluyveromyces marxianus* being CCTCC No: M 2023217.

The present application provides mutagenic *Kluyveromyces marxianus,* wherein the biomass of the mutagenic *Kluyveromyces marxianus* is increased by more than 5%, preferably more than 10%, and further preferably 10-30%, compared with the above-mentioned *Kluyveromyces marxianus.*

Preferably, for the mutagenic *Kluyveromyces marxianus,* the protein content of the mutagenic *Kluyveromyces marxianus* is increased by more than 5%, preferably more than 10%, and further preferably 15-25%, compared with the above-mentioned *Kluyveromyces marxianus* with the accession number of CCTCC No: M 2023217.

Preferably, for the *Kluyveromyces marxianus,* the accession number of the *Kluyveromyces marxianus* is CCTCC No: M 20222110.

Preferably, for the *Kluyveromyces marxianus,* the *Kluyveromyces marxianus* has tolerance to an organic acid, and preferably, the organic acid is lactic acid.

Preferably, for the *Kluyveromyces marxianus,* a tolerance concentration of the *Kluyveromyces marxianus* to the lactic acid is less than 5.5wt%, preferably 1-5.5wt%.

Preferably, for the *Kluyveromyces marxianus,* the *Kluyveromyces marxianus* has tolerance to a low pH value.

Preferably, for the *Kluyveromyces marxianus,* the *Kluyveromyces marxianus* uses an organic acid as a carbon source.

The present application provides a use of the above-mentioned *Kluyveromyces marxianus* in the production of bacteria proteins.

The present application provides a use of the above-mentioned *Kluyveromyces marxianus* in high-value transformation of an agricultural and forestry product, and preferably, the agricultural and forestry product is corn steep liquor.

The present application provides a microbial agent. The microbial agent includes the above-mentioned *Kluyveromyces marxianus.*

The present application provides a fermentation composition, which is obtained by fermenting the above-mentioned *Kluyveromyces marxianus.*

The application provides a method for producing a bacteria protein. The method includes: fermenting the above-mentioned *Kluyveromyces marxianus* or the above-mentioned microbial agent to obtain the bacteria protein.

The present application provides a food. The food contains the above-mentioned fermentation composition or the bacteria protein obtained by the above-mentioned method.

The present application provides feed. The feed contains the above-mentioned fermentation composition or the bacteria protein obtained by the above-mentioned method.

The present application provides a use of the above-mentioned original strain or mutagenic strain in feed production.

The present application has the following beneficial effects.

The AMCC 30634 strain and the AMCC 31342 strain of the present application have low pH tolerance, lactic acid tolerance and utilization capacity, and can effectively transform lactic acid into bacteria and bacteria proteins. In particular, the mutagenic AMCC 31342 strain can tolerate lactic acid having a pH of 2.0 and a concentration of 5%, and has tolerance up to 80-90% under the condition that the concentration of the lactic acid is 4.5-5.5%, further indicating that this strain has more efficient lactic acid utilization capacity in corn steep liquor fermentation, can increase the additional value of the corn industry, achieves high-value utilization of low biomass resources, reduces, as an alternative protein, the pressure on environmental resources caused by the traditional breeding industry, relieve the protein requirements of global population and the field of feed, and achieves economic development and environmental protection of the fermentation industry.

**Strain preservation information**

The *Kluyveromyces marxianus* strain used in the present application is preserved in the China Center for Type Culture Collection (CCTCC) on February 27, 2023, the accession number is CCTCC No: M 2023217, the accession address is Wuhan University, Wuhan, China, the postal code is 430072, and the telephone is 027-68754052.

The *Kluyveromyces marxianus* obtained by mutagenesis in the present application is preserved in the China Center for Type Culture Collection (CCTCC) on December 30, 2022, the accession number is CCTCC No: M 20222110, the accession address is Wuhan University, Wuhan, China, the postal code is 430072, and the telephone is 027-68754052.

**Brief Description of the Drawings**

FIG. 1 shows a schematic diagram of a colony morphology of *Kluyveromyces marxianus.*

FIG. 2 shows a schematic diagram of a morphology of *Kluyveromyces marxianus* under a microscope.

FIG. 3 is a schematic diagram of growth curves of a mutagenic strain and an original strain at different pH values.

FIGS. 4A to 4C are schematic diagrams of growth curves of the mutagenic strain and the original strain in YPD mediums containing different concentrations of lactic acids, wherein FIG. 4A is a schematic diagram of growth curves of the mutagenic strain and the original strain in a YPD medium containing 2% lactic acid, FIG. 4B is a schematic diagram of growth curves of the mutagenic strain and the original strain in a YPD medium containing 4% lactic acid, and FIG. 4C is a schematic diagram of growth curves of the mutagenic strain and the original strain in a YPD medium containing 5% lactic acid.

FIG. 5 is a schematic diagram of growth curves of the mutagenic strain and the original strain in a carbon source medium containing 2% lactic acid.

FIG. 6 is a schematic diagram of growth curves of the mutagenic strain and the original strain in a corn steep liquor-simulated fermentation medium.

**Detailed Description of the Invention**

The present application provides *Kluyveromyces marxianus,* wherein the accession number of the *Kluyveromyces marxianus* is CCTCC No: M 2023217.

The above-mentioned strain of the present application is a strain which is isolated from samples of fermented dairy products in the Tibet Autonomous Region. This strain has excellent resistance to an organic acid and can grow using lactic acid as a carbon source. In addition, this strain has low pH tolerance.

In the present application, the above-mentioned strain with the accession number of CCTCC No: M 2023217 is subjected to mutagenesis as an original strain to obtain a strain with better performances. In the present application, a method for mutagenesis is not limited in the present application in any way. A person skilled in the art can choose conventional methods for mutagenesis. For example, an ARTP mutagenesis method may be used for mutagenesis. That is, strains may be subjected to mutagenesis at different times to screen the strains.

The original strain refers to an originating strain used for breeding treatment. In the present application, the above-mentioned strain with the accession number of CCTCC No: M 2023217 is subjected to mutagenesis as an original strain to obtain a mutagenic strain with better performances.

In the present application, an ITS sequence of the above-mentioned strain with the accession number of CCTCC No: M 2023217 is shown in SEQ ID NO: 3 as follows:

CCAACGGGGATTGCCTTAGTACGGCGAGTGAAGCGGCAAAAGCTCAAATTTGAAATCT

GGCGTCTTCGACGTCCGAGTTGTAATTTGAAGAAGGCGACTTTGTAGCTGGTCCTTGTC

TATGTTCCTTGGAACAGGACGTCATAGAGGGTGAGAATCCCGTGTGGCGAGGATCCCA

GTTATTTGTAAAGTGCTTTCGACGAGTCGAGTTGTTTGGGAATGCAGCTCTAAGTGGGT

GGTAAATTCCATCTAAAGCTAAATATTGGCGAGAGACCGATAGCGAACAAGTACAGTGA

TGGAAAGATGAAAAGAACTTTGAAAAGAGAGTGAAAAAGTACGTGAAATTGTTGAAA

GGGAAGGGCATTTGATCAGACATGGCGTTTGCTTCGGCTTTCGCTGGGCCAGCATCAG

TTTTAGCGGTTGGATAAATCCTCGGGAATGTGGCTCTGCTTCGGTAGAGTGTTATAGCC

CGTGGGAATACAGCCAGCTGGGACTGAGGATTGCGACTTTTGTCAAGGATGCTGGCGT

AATGGTTAAATGCCGCCCGTCTTGAACCCACGGACCA.

In the present application, the ITS sequence refers that in an rDNA gene, an intergenic transcribed spacer of 5.8S rDNA and 28S rDNA is called ITS whose length and sequence change greatly, and an amplification product of this sequence is subjected to RFLP or sequence analysis, and may be used for classification and identification of different biotypes, strains, species, genera of fungi, and even differentiation of species that are very closely related.

The present application provides mutagenic *Kluyveromyces marxianus.* The biomass of the mutagenic *Kluyveromyces marxianus* is increased by more than 5%, preferably more than 10%, and further preferably 10-30%, and the protein content of the mutagenic *Kluyveromyces marxianus* is increased by more than 5%, preferably more than 10%, and further preferably 15-25%, compared with *Kluyveromyces marxianus* with the accession number of CCTCC No: M 2023217.

In some embodiments, the *Kluyveromyces marxianus* has tolerance to an organic acid, and preferably, the organic acid is lactic acid.

In the present application, the tolerance refers to a growth condition of a strain in the presence of a substance. For example, in the case of lactic acid, the tolerance refers to a growth condition of *Kluyveromyces marxianus* in the presence of lactic acid.

In some embodiments, a tolerance concentration of the *Kluyveromyces marxianus* to the lactic acid is less than 5%, preferably 1-5%.

For example, the tolerance concentration of the *Kluyveromyces marxianus* to the lactic acid is 5%, 4.5%, 4%, 3.5%, 2%, 2.5%, 1.5%, 1%, etc.

In the present application, both the original strain and the mutagenic strain have better tolerance to the lactic acid and have a tolerance concentration of 1-5.5wt%. The mutagenic strain has better tolerance to the lactic acid, and may have a tolerance concentration up to 5.5wt%. In some embodiments, the tolerance of the mutagenic *Kluyveromyces marxianus* strain is 80-90%, for example, may reach 80%, 81%, 82%, 83%, 84%, 85%, 86%, 87%, 88%, 89%, or 90%, under the condition that the lactic acid concentration is 4.5-5.5wt%.

In some embodiments, the accession number of the mutagenic *Kluyveromyces marxianus* strain is CCTCC No: M 20222109.

In some embodiments, the *Kluyveromyces marxianus* has tolerance to a low pH value.

In the present application, the low pH refers to a pH value below 4.0.

In the present application, the original strain and the mutagenic strain have better tolerance to a low pH value, e.g., below 4.0. The mutagenic strain has better tolerance to low pH and can grow under the condition that pH is 2, indicating that the original strain and the mutagenic strain of the present application both have better tolerance to low pH.

In some embodiments, the *Kluyveromyces marxianus* uses an organic acid as a carbon source. The present application provides a use of the above-mentioned *Kluyveromyces marxianus* with the accession number of CCTCC No: M 2023217 or the mutagenic *Kluyveromyces marxianus* in the production of bacteria proteins.

The *Kluyveromyces marxianus* with the accession number of CCTCC No: M 2023217 or the mutagenic *Kluyveromyces marxianus* of the present application may be used in the production of bacteria proteins due to high protein yield.

The present application provides a use of the above-mentioned *Kluyveromyces marxianus* with the accession number of CCTCC No: M 2023217 or the mutagenic *Kluyveromyces marxianus* in high-value transformation of an agricultural and forestry product. Preferably, the agricultural and forestry product is corn steep liquor.

The present application provides a microbial agent. The microbial agent contains the above-mentioned *Kluyveromyces marxianus* with the accession number of CCTCC No: M 2023217 or the mutagenic *Kluyveromyces marxianus.*

The present application provides a fermentation composition. The fermentation composition is obtained by fermenting the above-mentioned *Kluyveromyces marxianus* with the accession number of CCTCC No: M 2023217 or the mutagenic *Kluyveromyces marxianus.*

In the present application, fermentation conditions are not limited in the present application in any way. Fermentation is performed under conventional fermentation conditions in the art. For example, the fermentation can be performed at 25-37°C for 11-60 h.

The present application provides a method for producing a bacteria protein. The method includes: fermenting the above-mentioned *Kluyveromyces marxianus* with the accession number of CCTCC No: M 2023217 or the mutagenic *Kluyveromyces marxianus* or the above-mentioned microbial agent.

Fermentation conditions are not limited in the present application in any way. Fermentation is performed under conventional fermentation conditions in the art. For example, the fermentation can be performed at 25-37°C for 11-60 h.

The present application provides a food. The food contains the above-mentioned fermentation composition or the bacteria protein obtained by the above-mentioned method.

The present application provides feed. The feed contains the above-mentioned fermentation composition or the bacteria protein obtained by the above-mentioned method.

The present application provides a use of the above-mentioned original strain or mutagenic strain in feed production.

**Examples**

The manufacturers of the raw materials and equipment used in the present examples, as well as the equipment and analytical methods used in the analysis of products, are described as follows. The chemical substances which are not labeled are all chemically pure grades of conventional reagents. Table 1 is a reagent information table used in the examples. Table 2 is an instrument information table used in the examples.

Table 1 Reagent information table

| Reagent | Manufacturer |
| --- | --- |
| Yeast extract powder | Angel Yeast Co., Ltd. |
| Peptone | Angel Yeast Co., Ltd. |
| Glucose | Sinopharm Chemical Reagent Co., Ltd. HuShi Reagents |
| Xylose | Sinopharm Chemical Reagent Co., Ltd. HuShi Reagents |
| Cellobiose | Sinopharm Chemical Reagent Co., Ltd. HuShi Reagents |
| Lactic acid | Sinopharm Chemical Reagent Co., Ltd. HuShi Reagents |
| Agar | Beijing Solarbio Science & Technology Co., Ltd. |

Table 2 Instrument information table

| Instrument | Model | Manufacturer |
| --- | --- | --- |
| Analytical balance | ME4002E | METTLER TOLEDO |
| pH meter | PB-10 | Sartorius |
| Superclean bench | SKJH-1109 | Shanghai Sukun Industry Co., Ltd. |
| Constant temperature shaker | ZWYR-2102C | Shanghai Zhicheng Analytical Instrument Manufacturing Co., Ltd. |
| Biochemical incubator | SPX-158L | Ningbo Scientz Biotechnology Co., Ltd. |

(continued)

| Instrument | Model | Manufacturer |
|---|---|---|
| Centrifuge | DL-5200B-II | Shanghai Anting Scientific Instrument Factory |
| Optical microscope | CX43 | OLYMPUS |
| Fully automatic growth curve analyzer | BioscreenC | OY Growth Curves |
| Electrothermal blowing dry box | DHG-9070A | Shanghai Jinghong Experimental Equipment Co., Ltd. |

Furthermore, the mediums involved in the following examples were as follows.

YPD medium: 10 g of yeast extract powder, 20 g of glucose, 20 g of peptone, and 1000 mL of water, having pH of 4.8, all of which were sterilized at 115°C for 20 min.

Lactic acid-carbon source medium: 10 g of yeast extract powder, 20 g of peptone, and 1000 mL of water, having pH of 4.8, all of which were sterilized at 115°C for 20 min. Lactic acid was filtered, sterilized and then added to the above-mentioned medium.

Corn steep liquor-simulated medium: 10 g of yeast extract powder, 20 g of peptone, 3 g of fructose, 64.5 g of glucose, 1.5 g of glycerin, 1 g of acetic acid, 30 g of lactic acid, and 1000 mL of water, having pH of 4.8, all of which were sterilized at 115°C for 15 min.

**Example 1**

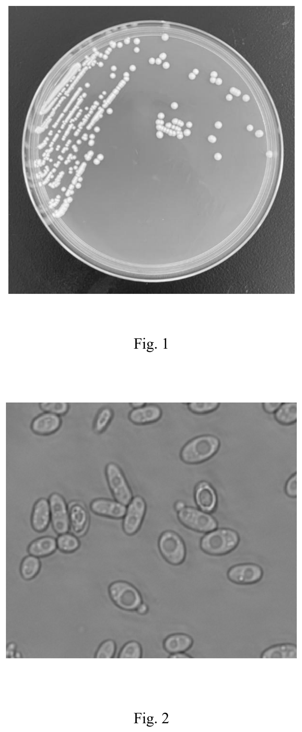

Each fermented dairy product sample collected in Xizang Autonomous Region was dissolved in sterile water and mixed well. A bacterial suspension was pipetted and diluted in 10-fold series to prepare a $10^{-5}$ bacterial suspension and a $10^{-6}$ bacterial suspension, which were then coated in a YPD medium and cultured at 30°C for 24-48 h. A yeast morphology was observed under a microscope, and the results were shown in FIG. 1; and meanwhile, the characteristics of single colonies on a flat plate were observed, and the results were shown in FIG. 2. It can be seen from FIG. 1 and FIG. 2, the colonies of this yeast strain had cheese-like character, milky white color, smooth surfaces, neat edges, and oval micromorphology, showing budding and reproductive properties. This yeast strain was subjected to streak purification twice, inoculated into a YPD slant medium, and stored at 4°C. A yeast strain genome was extracted.

The ITS sequences of the yeast were amplified with ITS1 (5'-TCCGTAGGTGAACCTGCGG- 3', SEQ ID NO: 1) and ITS4 (5'-TCC TCCGCTTATTGATATGC- 3', SEQ ID NO: 2) as primers. After 1% gel electrophoresis detection and sequencing, the sequences were compared with those on GenBank by Blast analysis, and the sequences with a similarity greater than 99% were considered to be from the same species. In conjunction with morphological analysis and molecular identification, this strain was *Kluyveromyces marxianus* with a strain number of AMCC30634. AMCC was the abbreviation of Angel Microbiological Culture Collection Center, and its ITS sequence was shown in SEQ ID NO: 3. This strain was preserved in the China Center for Type Culture Collection (CCTCC) on February 27, 2023, and the accession number was CCTCC No: M 2023217.

Example 2 Mutagenesis of *Kluyveromyces marxianus*

The strain AMCC 30634 obtained in Example 1 was inoculated at an inoculation amount of 2% into a test tube filled with 5 mL of YPD liquid, and cultured at 30°C, 180 rpm for 20 h. Bacteria were collected by centrifugation with a centrifuge, washed once with ddH$_2$O, and then resuspended to a viable count of approximately $5 \times 10^7$ CFU/mL. 10 μL of bacteria was pipetted, dropped to a metal plate and then coated well. The mutagenesis conditions were as follows: a treatment distance was 2 mm, a treatment power was 120 W, an air flow rate was 10 SLM, a dispensing volume was 10 μL, and the irradiation times were 0, 5, 10, 15, 20, 25, and 30 s, respectively. Then, the bacteria was put in 1 mL of normal saline, mixed well and subjected to gradient dilution. 0.1 mL of bacteria was then pipetted, coated in a YPD solid medium, and cultured at 30°C for 48 h. A lethality rate was determined according to the conventional methods in the art. From a flat plate with a mortality rate of over 98%, 98 single colonies with larger size than the original strain were picked out and inoculated into a YPD medium for activation. The 98 strains were all mutagenic strains, named AMCC 31342, and were graded and screened according to Example 3 to Example 8 to obtain a dominant strain. This strain was preserved in the China Center for Type Culture Collection (CCTCC) on December 30, 2022, and the accession number was CCTCC No: M 20222110.

Example 3 pH tolerance analysis

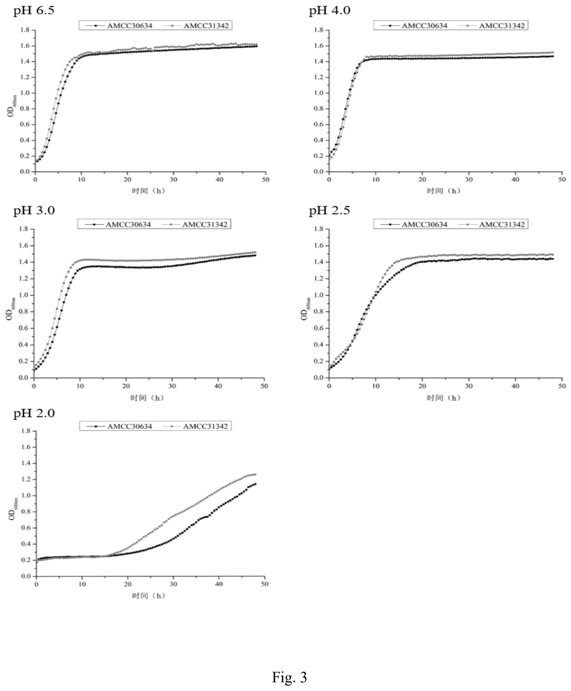

The activated bacterial solution of the mutagenic strain obtained in Example 2 was inoculated at an inoculation amount of 3% into a 100-well culture plate filled with 300 μL of YPD mediums with different pHs (2.0, 2.5, 3.0, 4.0, 6.5), and a BioscreenC instrument was prepared for on-machine determination. The following parameters were set: the temperature was 30°C, the time was 24 h, the wavelength was 600 nm, the data was measured every 30 min, followed by determination of growth curves, and the results were shown in FIG. 3. A specific growth rate and tolerance were determined, and the results were shown in Table 3. The specific growth rate and tolerance were calculated according to the following formulas:

$$\text{specific growth rate } (\mu) = \frac{LNOD2 - LNOD1}{t2 - t1}$$

OD1: a corresponding $OD_{600}$ value at t1;
OD2: a corresponding $OD_{600}$ value at t2;
t2: end time of a logarithmic growth phase; and
t1: start time of the logarithmic growth phase;

$$\text{pH tolerance } (\%) = \frac{OD2 - OD1}{OD4 - OD3} \times 100\%$$

$OD_1$-a corresponding $OD_{600}$nm value of a strain in a YPD medium at certain pH at 0 h;
$OD_2$-a corresponding $OD_{600}$nm value of a strain in a YPD medium at certain pH at 48 h;
$OD_3$-a corresponding $OD_{600}$nm value of a strain in a YPD medium (natural pH of 6.5) at 0 h;
$OD_4$-a corresponding $OD_{600}$nm value of a strain in a YPD medium (natural pH of 6.5) at 48 h; and

$$\text{relative percentage } (\%) = \frac{\text{Content of a physiological index in screened strain}}{\text{Content of a physiological index in control strain}} \times 100\%.$$

Table 3 Growth indexes of strains at different pHs

| pH | Strain | $OD_{max}$(nm) | Relative percentage (%) | $\mu_{max}$(h$^{-1}$) | Relative percentage (%) | pH tolerance (%) | Relative percentage (%) |
|---|---|---|---|---|---|---|---|
| Natural pH (6.5) | AMCC 30634 | 1.595 | | 0.485 | | - | |
| | AMCC 31342 | 1.619 | 102 | 0.535 | 110 | - | |
| 2 | AMCC 30634 | 1.141 | | 0.08 | | 72 | |
| | AMCC 31342 | 1.237 | 108 | 0.116 | 145 | 76 | 106 |
| 2.5 | AMCC 30634 | 1.44 | | 0.371 | | 90 | |
| | AMCC 31342 | 1.492 | 104 | 0.439 | 118 | 92 | 102 |
| 3 | AMCC 30634 | 1.483 | | 0.442 | | 93 | |
| | AMCC 31342 | 1.521 | 103 | 0.457 | 103 | 94 | 101 |
| 4 | AMCC 30634 | 1.468 | | 0.462 | | 92 | |
| | AMCC 31342 | 1.517 | 103 | 0.487 | 105 | 94 | 102 |

As can be seen from FIG. 3 and Table 3, both strains could grow at pH of 2.0-6.5, and had tolerance that was higher than 90% under the condition that pH was 2.5-6.5. However, both strains grew slowly under the condition that pH was 2.0. Under this condition, the tolerance of the strain AMCC 30634 was 72%, and the tolerance of the mutagenic strain AMCC 31342 was 76%, which was 6% higher than that of the original strain. In practical applications, a large amount of lactic acid would be produced after fermentation of low biomass resources, resulting in decrease in pH in an environment, further indicating that the strains AMCC 30634 and AMCC 31342 could grow and produce a large number of bacteria in a low pH environment. In addition, the strain AMCC 31342 had a higher potential to achieve high-value utilization of low biomass resources, and at the same time, could reduce the cost increase caused by the use of an acidic neutralizer.

Example 4 Screening of lactic acid tolerance

Components of corn steep liquor were detected by HPLC (after the corn steep liquor was diluted, an ion-exchange chromatography column at a column temperature of 65°C was selected, and the components of the corn steep liquor were detected by using a differential refractive index detector as detection means). The contents of the main components were shown in Table 4. The results showed that the main carbon source in the corn steep liquor was lactic acid which had the content of about 8%. The mutagenic strain was screened for tolerance with 0%, 2%, 4%, 5%, and 6% lactic acids.

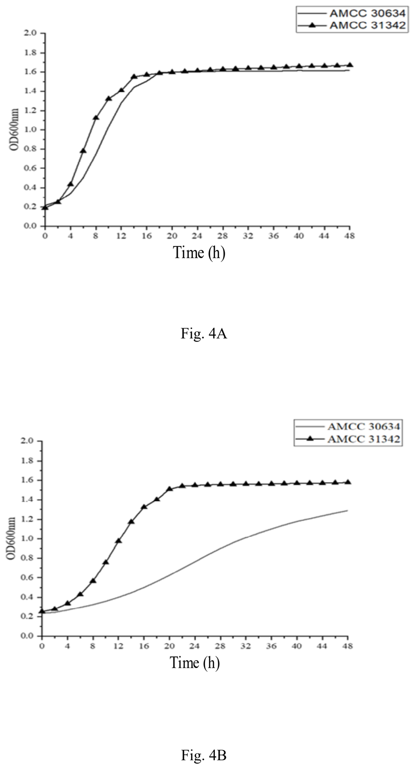

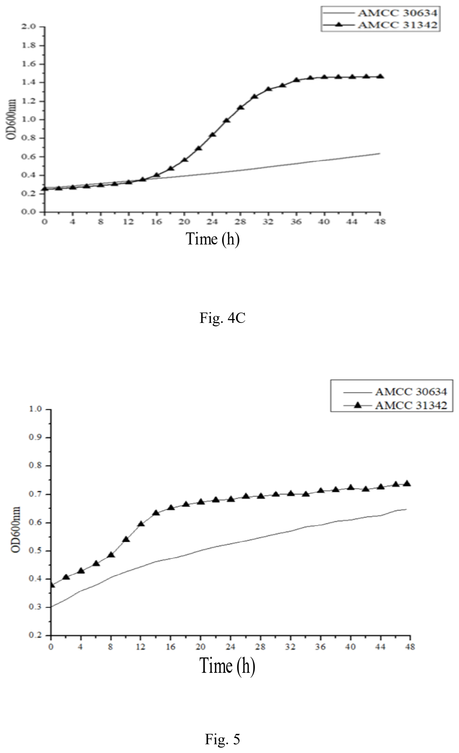

The activated bacterial solution of the mutagenic strain obtained in Example 2 was inoculated at an inoculation amount of 3% into a 100-well culture plate filled with 300 $\mu$LL of YPD mediums with different lactic acid concentrations, and a BioscreenC instrument was prepared for on-machine determination. The following parameters were set: the temperature was 30°C, the time was 24 h, the wavelength was 600 nm, the data was measured every 30 min, followed by determination of growth curves, and the results were shown in FIG. 4A to FIG. 4C. FIG. 4A was a schematic diagram of growth curves of the two strains in a YPD medium containing 2% lactic acid, FIG. 4B is a schematic diagram of growth curves of the two strains in a YPD medium containing 4% lactic acid, and FIG. 4C is a schematic diagram of growth curves of the two strains in a YPD medium containing 5% lactic acid. OD600nm, a specific growth rate ($\mu$), a lag phase, a tolerance and a relative percentage were calculated, wherein formulas for calculating the specific growth rate and the relative percentage were the same as those in Example 3, the tolerance was calculated according to the following formula, and the results were shown in Table 5 to Table 8.

$$\text{Lactic acid tolerance (\%)}= \frac{OD2-OD1}{OD4-OD3}\times 100\%$$

$OD_1$-a corresponding $OD_{600}$nm value of a strain in a YPD medium at certain lactic acid concentration at 0 h;
$OD_2$-a corresponding $OD_{600}$nm value of a strain in a YPD medium at certain lactic acid concentration at 48 h;
$OD_3$-a corresponding $OD_{600}$nm value of a strain in a YPD medium at 0 h; and
$OD_4$-a corresponding $OD_{600}$nm value of a strain in a YPD medium at 48 h.

Table 4 Content of main components in corn steep liquor (%)

| Components | Lactic acid | Total sugar | Reducing sugar |
|---|---|---|---|
| Content | 8 | 3 | 2.7 |

Table 5 Growth indexes of strains at lactic acid concentration of 0% (common YPD)

| Strain | OD600 (nm) | Relative percentage (%) | $\mu$(h$^{-1}$) | Relative percentage (%) | Lag phase (h) | Relative percentage (%) |
|---|---|---|---|---|---|---|
| AMCC 30634 | 1.695 | - | 0.210 | - | 3.4 | - |
| AMCC 31342 | 1.757 | 104 | 0.357 | 170 | 2 | 59 |

Table 6 Growth indexes of strains at lactic acid concentration of 2% (pH of 3.06)

| Strain | OD600 (nm) | Relative percentage (%) | $\mu$ (h$^{-1}$) | Relative percentage (%) | Lag phase (h) | Relative percentage (%) | Lactic acid tolerance (%) | Relative percentage (%) |
|---|---|---|---|---|---|---|---|---|
| AMCC 30634 | 1.616 | - | 0.210 | - | 4 | - | 95 | - |

(continued)

| Strain | OD600 (nm) | Relative percentage (%) | μ (h⁻¹) | Relative percentage (%) | Lag phase (h) | Relative percentage (%) | Lactic acid tolerance (%) | Relative percentage (%) |
|---|---|---|---|---|---|---|---|---|
| AMCC 31342 | 1.877 | 116 | 0.316 | 150 | 2 | 50 | 107 | 113 |

Table 7 Growth indexes of strains at lactic acid concentration of 4% (pH of 2.72)

| Strain | OD600 (nm) | Relative percentage (%) | μ (h⁻¹) | Relative percentage (%) | Lag phase (h) | Relative percentage (%) | Lactic acid tolerance (%) | Relative percentage (%) |
|---|---|---|---|---|---|---|---|---|
| AMCC 30634 | 1.289 | - | 0.064 | - | 7.4 | - | 76 | - |
| AMCC 31342 | 1.577 | 122 | 0.150 | 234 | 4 | 54 | 90 | 118 |

Table 8 Growth indexes of strains at lactic acid concentration of 5% (pH of 2.63)

| Strain | OD600 (nm) | Relative percentage (%) | μ (h⁻¹) | Relative percentage (%) | Lag phase (h) | Relative percentage (%) | Lactic acid tolerance (%) | Relative percentage (%) |
|---|---|---|---|---|---|---|---|---|
| AMCC 30634 | 0.636 | - | 0.034 | - | 16 | - | 38 | - |
| AMCC 31342 | 1.464 | 230 | 0.103 | 303 | 12 | 75 | 83 | 218 |

As can be seen from the results of FIG. 4A to FIG. 4C and Table 5 to Table 8, both AMCC 30634 and AMCC 31342 had tolerance to lactic acid and had a phenomenon of secondary growth. The maximum tolerance concentration of lactic acid was 5% lactic acid (an OD600 increase of more than 50% was considered to be growth); and if the lactic acid concentration was greater than 5%, the growth of yeast strains was inhibited. When the lactic acid concentration was 0-5%, the biomass (OD600) and specific growth rate of the mutagenic strain AMCC 31342 were both higher than those of the original strain AMCC 30634, and the lag phases of the mutagenic strain AMCC 31342 and the original strain AMCC 30634 were both advanced. When the lactic acid concentration was 2%, the tolerance of the AMCC 31342 strain was greater than 100%, indicating that the lactic acid concentration of 2% could promote its growth without any inhibiting effect, which was the optimal lactic acid growth concentration of the strain and could be applied to the actual fermentation production. When the lactic acid concentration was 5%, the biomass of the AMCC 31342 strain was increased by 130%, the specific growth rate was increased by 203%, the lag phase was shortened by 25% and the tolerance was increased by 118% compared with AMCC 30634.

Example 5 Analysis of lactic acid utilization and growth

The mutagenic strain and the original strain obtained in Example 2 were respectively inoculated into a 100-well culture plate filled with 300 μL of 2% lactic acid-carbon source medium, and a BioscreenC instrument was prepared for on-machine determination. The following parameters were set: the temperature was 30°C, the time was 24 h, the wavelength was 600 nm, the data was measured every 30 min, followed by determination of growth curves, and the results were shown in FIG. 5. The OD600nm was calculated, the specific growth rate (μ) and relative percentage were calculated according to the formula in Example 3, and the lag phase were calculated. The results were shown in Table 9.

Table 9 Growth indexes of strains under 2% lactic acid-carbon source medium

| Strain | OD600 (nm) | Relative percentage (%) | μ(h⁻¹) | Relative percentage (%) | Lag phase (h) | Relative percentage (%) |
|---|---|---|---|---|---|---|
| AMCC 30634 | 0.647 | - | 0.067 | - | 8 | - |
| AMCC 31342 | 0.737 | 114 | 0.069 | 103 | 6 | 75 |

As can be seen from FIG. 5 and Table 9, both AMCC 30634 and AMCC 31342 strains could use lactic acid as a carbon source for growth, and the biomass of the mutagenic strain AMCC 31342 was increased by 14%, the specific growth rate was increased by 3% and the lag phase was shortened by 25% compared with AMCC 30634, further indicating that after mutagenesis, the lactic acid utilization capacity of AMCC 31342 was improved, and AMCC 31342 can be transformed into bacteria using lactic acid.

Example 6 Small-system screening of corn steep liquor-simulated medium (screening in 100-well culture plate)

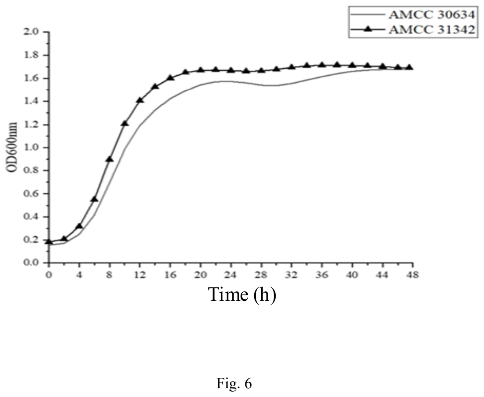

The mutagenic strain and the original strain obtained in Example 2 were respectively inoculated at an inoculation amount of 3% into a 100-well culture plate filled with 300 μL of corn steep liquor-simulated medium, and a BioscreenC instrument was prepared for on-machine determination. The following parameters were set: the temperature was 30°C, the time was 24 h, the wavelength was 600 nm, the data was measured every 30 min, followed by determination of growth curves, and the results were shown in FIG. 6. The OD600nm was calculated, the specific growth rate ($\mu$) and relative percentage were calculated according to the formula in Example 3, and the lag phase were calculated. The results were shown in Table 10.

Table 10 Growth indexes of strains under corn steep liquor-simulated medium

| Strain | OD600 (nm) | Relative percentage (%) | $\mu(h^{-1})$ | Relative percentage (%) | Lag phase (h) | Relative percentage (%) |
|---|---|---|---|---|---|---|
| AMCC 30634 | 1.679 | - | 0.276 | - | 4 | - |
| AMCC 31342 | 1.692 | 101 | 0.290 | 105 | 3.5 | 88 |

As can be seen from FIG. 6 and Table 10, the AMCC 30634 and AMCC 31342 strains could grow normally in a corn steep liquor system, and the specific growth rate of the mutagenic strain AMCC 31342 was increased by 5% and the lag phase was shortened by 12% compared with AMCC 30634, indicating that AMCC 31342 entered the logarithmic growth phase faster and had more efficient utilization efficiency of corn steep liquor.

Example 7 Shake flask fermentation test of corn steep liquor-simulated medium

The mutagenic strain and the original strain obtained in Example 2 were inoculated at an inoculation amount of 0.5% into a shake flask filled with 300 mL of corn steep liquor-simulated medium, shaken at 30°C, cultured overnight, and centrifugally washed to obtain yeast milk. A rapid moisture analyzer was used to perform moisture detection of the yeast milk, a dry weight (the biomass was a weight of the yeast milk after centrifugation, and could be weighed directly: dry weight (g/L) = biomass (g/L) * (1-moisture%) of the yeast milk was calculated, and the relative percentage was calculated according to the calculation formula in Example 3. The results were shown in Table 11. A method for determining a protein was as follows:
1 g of yeast milk (accurate to 0.0002 g) was weighed accurately and placed into a digestive tube, added with 2.5 g of digestive powder, slowly added with 10 mL of concentrated sulfuric acid along a wall of the tube, then placed on a digestion device, and digested for about 3 h until it is smokeless; the solution became clear and light yellow, and then continued to be heated for 10 min. The digestive tube was taken out, placed for cooling, rinsed for the tube wall with about 30 mL of distilled water, cooled again, then transferred into a 100 mL volumetric flask, rinsed with a small amount of water for three times, poured into a volumetric flask, added with water to a constant volume scale, and shaken well to obtain a digestive solution for later use. 25 mL of the digestive solution was pipetted accurately into the digestive tube and placed on a distillation device. 25 mL of boric acid solution was added to a triangular flask, added with 4-6 drops of methyl red-bromocresol green mixed indicator as a receiving solution and placed on a receiving table. A circulating water valve was opened; 25 mL of sodium hydroxide solution was added to the digestive tube; a steam switch was switched on, and the receiving table was lift, such that a receiving tube was immersed in the receiving solution and distilled till the receiving solution was 150-200 mL; the receiving table was put down, and steam and circulating water was turned off; a receiving nozzle was rinsed with distilled water; and the receiving bottle was taken out. The receiving solution was titrated with a 0.05 mol/L sulfuric acid standard solution; an end point was reached as the color became reddish from green; a blank test was done at the same time according to the above method; and the protein content was calculated according to the following formula:

$$X = (C(V_1 - V_2) \times 0.01401)/(W \times Ds \times 25/100) \times 100 \times 6.25$$

in which: X represented a percentage of the protein in the sample, %;
C represented a concentration of the sulfuric acid standard solution, mol/L;

$V_1$ represented a volume of the sulfuric acid standard solution consumed by the titration of the sample, mL;
$V_2$ represented a volume of the sulfuric acid standard solution consumed by the titration of a control, mL;
0.01401 referred to a mass of nitrogen expressed in grams equivalent to 1.00 mL of sulfuric acid [C(1/2H2SO4)=1.000 mol/L] standard solution;
W represented a mass of the sample, g;
Ds represented a percentage of a dry substance in the sample, %; and
6.25 represented a conversion factor of nitrogen to crude protein.

Table 11 Fermentation indexes for different strains

| Strain | Biomass (g/L) | Relative percentage (%) | Dry weight (g/ L) | Relative percentage (%) | Protein (%) | Relative percentage (%) |
|---|---|---|---|---|---|---|
| AMCC 30634 | 10.48 | - | 2.96 | - | 51.0 | - |
| AMCC 31342 | 13.33 | 127 | 3.94 | 133 | 51.3 | 101 |

The results showed that the biomass, dry weight and protein content indexes of the mutagenic strain AMCC 30634 were higher than those of the original strain AMCC 30634. The biomass was increased by 27% compared with the original strain AMCC 30634, the dry weight was increased by 33% compared with the original strain AMCC 30634, and the protein content was increased by 1% compared with the original strain AMCC 30634, further indicating that AMCC 30634 could be produced normally in an acidic environment of corn steep liquor and could be efficiently transformed into bacteria and bacteria proteins using the corn steep liquor.

Example 8 Small-scale fermentation test of corn steep liquor

A small-scale fermentation test was carried out on AMCC 30634 and AMCC 31341.
(1) whole corn steep liquor fermentation: a domestic Baoxing 45 L fermenter was selected for fed-batch fermentation, and a fed-batch medium contained 100% of corn steep liquor, and was sterilized at 115°C for 20 min. Fermentation was performed for 14 h. The pH was controlled at 4.3-5.6 and the culture temperature was 30-32°C in the fermentation process. At the end of fermentation, fermentation broth was centrifuged, followed by suction filtration. The weight of the yeast milk was weighed and the moisture of the yeast milk was detected (with a rapid moisture detector). The dry weight of the yeast milk was calculated. The volume of the fermentation broth and the weight of corn steep liquor were measured. The fermentation indexes of the two strains were compared according to the following formula, and the results were shown in Table 12, wherein the unit consumption was defined as: grams of corn steep liquor required to produce 1 g of dry yeast bacteria/protein.

$$\text{Unit consumption of bacteria (g)} = \text{weight of corn steep liquor/dry weight of produced yeast}$$

$$\text{Unit consumption of protein (g)} = \text{unit consumption of bacteria} * \text{protein content}$$

(a protein measurement method was the same as that in Example 7)

$$\text{Bacteria concentration (g/L)} = \text{weight of yeast milk/empty fermenter volume}$$

$$\text{Bacteria protein concentration (g/L)} = \text{bacteria concentration} * \text{protein content}$$

$$\text{Bacteria protein (g)} = \text{dry weight} * \text{protein content}$$

Table 12 Small-scale fermentation indexes of whole corn steep liquor

| Strain | Unit consumption of bacteria (g) | Unit consumption of protein (g) | Empty fermenter volume (L) | Bacteria concentration (g/L) | Dry weight (g) | Protein content (%) | Bacteria protein concentration (g/L) | Bacteria protein (g) |
|---|---|---|---|---|---|---|---|---|
| AMCC 30634 | 38.37 | 67.08 | 21.8 | 125 | 558 | 53.8 | 67 | 300 |
| AMCC 31342 | 33.7 | 62.69 | 28 | 110 | 631 | 57.2 | 63 | 361 |

(2) Sugar-supplemented fermentation of corn steep liquor: a domestic Baoxing 45 L fermenter was selected for fed-batch fermentation, and a fed-batch medium contained 50% of corn syrup and 50% of glucose (in percentage by weight), and was sterilized at 115°C for 20 min. Fermentation was performed for 14 h. The pH was controlled at 4.3-5.6 and the culture temperature was 30-32°C in the fermentation process. The fermentation indexes of the two strains were compared, and the results were shown in Table 13. (Unit consumption: grams of carbon source required to produce 1g of dry yeast bacteria/protein.)

Table 13 Small-scale sugar-supplemented fermentation indexes of corn steep liquor

| Strain | Unit consumption of bacteria (g) | Unit consumption of protein (g) | Empty fermenter volume (L) | Bacteria concentration (g/L) | Dry weight (g) | Protein content (%) | Bacteria protein concentration (g/L) | Bacteria protein (g) dry |
|---|---|---|---|---|---|---|---|---|
| AMCC 30634 | 4.7 | 8.0 | 17.6 | 310 | 1118 | 58.9 | 183 | 659 |
| AMCC 31342 | 4.4 | 7.3 | 19 | 300 | 1169 | 60 | 180 | 701 |

In a fermentation system of whole corn steep liquor, lactic acid was used as a main carbon source, and the two strains could grow normally, indicating that the AMCC 31342 and AMCC 31342 strains had tolerance to the lactic acid and could be transformed into bacteria and bacteria proteins by using lactic acid. The unit consumption of bacteria and the unit consumption of protein were compared. The AMCC 31342 strain had more efficient lactic acid utilization capacity, the dry weight of the mutagenic strain AMCC 31342 was increased by 13% compared with the original strain, and the bacteria protein of the mutagenic strain AMCC 31342 was increased by 20% compared with the original strain.

In a sugar-supplemented fermentation system of corn steep liquor, glucose and lactic acid were used as a carbon source, AMCC 31342 had lower unit consumption, the dry weight index of AMCC 31342 was increased by 5% compared with the original strain, and the bacteria protein of AMCC 31342 was increased by 7% compared with the original strain. The dry weight and protein indexes of the two strains were higher than those in whole corn steep liquor fermentation, indicating that the production efficiency was higher after sugar supplementation, that is, there was still some room for optimization in the fermentation process.

It was further explained that the two strains in the present invention could achieve high-value utilization of low-cost biomass, and were used in the field of foods or feed proteins as alternative proteins to reduce the pressure on environmental resources brought by the traditional aquaculture industry, alleviate the protein requirements of global population and the field of feed, and achieve the economic development and environmental protection of the fermentation industry.

The foregoing descriptions are merely preferred examples of the present invention, and are not intended to limit the present invention in any form. Any modifications, equivalent substitutions, improvements, etc. made within the spirit and principles of the present invention should fall within the protection scope of the present invention.

Sequence listings

| SEQ ID NOs: | Sequence description | Sequences |
|---|---|---|
| SEQ ID NO: 1 | Primer sequence | TCCGTAGGTGAACCTGCGG |
| SEQ ID NO: 2 | Primer sequence | TCC TCCGCTTATTGATATGC |
| SEQ ID NO: 3 | ITS sequence of original strain | CCAACGGGGATTGCCTTAGTACGGCGAGTGAAGCGGCAAAA GCTCAAATTTGAAATCTGGCGTCTTCGACGTCCGAGTTGTAA TTTGAAGAAGGCGACTTTGTAGCTGGTCCTTGTCTATGTTCCT TGGAACAGGACGTCATAGAGGGTGAGAATCCCGTGTGGCGA GGATCCCAGTTATTTGTAAAGTGCTTTCGACGAGTCGAGTTG TTTGGGAATGCAGCTCTAAGTGGGTGGTAAATTCCATCTAAA GCTAAATATTGGCGAGAGACCGATAGCGAACAAGTACAGTGA TGGAAAGATGAAAAGAACTTTGAAAAGAGAGTGAAAAAGT ACGTGAAATTGTTGAAAGGGAAGGGCATTTGATCAGACATG GCGTTTGCTTCGGCTTTCGCTGGGCCAGCATCAGTTTTAGCG GTTGGATAAATCCTCGGGAATGTGGCTCTGCTTCGGTAGAGT GTTATAGCCCGTGGGAATACAGCCAGCTGGGACTGAGGATTG CGACTTTTGTCAAGGATGCTGGCGTAATGGTTAAATGCCGCC CGTCTTGAACCCACGGACCA |

## Claims

1. *Kluyveromyces marxianus,* the accession number of the *Kluyveromyces marxianus* being CCTCC No: M 2023217.

2. Mutagenic *Kluyveromyces marxianus,* **characterized in that** the biomass of the mutagenic *Kluyveromyces marxianus* is increased by more than 5%, preferably more than 10%, and further preferably 10-30%, compared with the *Kluyveromyces marxianus* according to claim 1.

3. The *Kluyveromyces marxianus* according to claim 2, **characterized in that** the protein content of the mutagenic *Kluyveromyces marxianus* is increased by more than 5%, preferably more than 10%, and further preferably 15-25%, compared with the *Kluyveromyces marxianus* according to claim 1.

4. The *Kluyveromyces marxianus* according to claim 2 or 3, **characterized in that** the accession number of the *Kluyveromyces marxianus* is CCTCC No: M 20222110.

5. The *Kluyveromyces marxianus* according to any one of claims 1 to 4, **characterized in that** the *Kluyveromyces marxianus* has tolerance to an organic acid, and preferably, the organic acid is lactic acid.

6. The *Kluyveromyces marxianus* according to claim 5, **characterized in that** a tolerance concentration of the *Kluyveromyces marxianus* to the lactic acid is less than 5.5wt%, preferably 1-5.5wt%.

7. The *Kluyveromyces marxianus* according to any one of claims 1 to 6, **characterized in that** the *Kluyveromyces marxianus* has tolerance to a low pH value.

8. The *Kluyveromyces marxianus* according to any one of claims 1 to 7, **characterized in that** the *Kluyveromyces marxianus* uses an organic acid as a carbon source.

9. A use of the *Kluyveromyces marxianus* according to any one of claims 1 to 8 in production of bacteria proteins.

10. A use of the *Kluyveromyces marxianus* according to any one of claims 1 to 8 in the high-value transformation of an agricultural and forestry product, and preferably, the agricultural and forestry product is corn steep liquor.

11. A microbial agent, containing the *Kluyveromyces marxianus* according to any one of claims 1 to 8.

12. A fermentation composition, which is obtained by fermenting the *Kluyveromyces marxianus* according to any one of claims 1 to 8.

13. A method for producing a bacteria protein, comprising:
    fermenting the *Kluyveromyces marxianus* according to any one of claims 1 to 8 or the microbial agent according to claim 11 to obtain the bacteria protein.

14. A food, containing the fermentation composition according to claim 12 or the bacteria protein obtained by the method according to claim 13.

15. A feed, containing the fermentation composition according to claim 12 or the bacteria protein obtained by the method according to claim 13.

16. A use of the *Kluyveromyces marxianus* according to any one of claims 1 to 8 in production of feed.

Fig. 1

Fig. 2

Fig. 3

Fig. 4A

Fig. 4B

Fig. 4C

Fig. 5

Fig. 6

## INTERNATIONAL SEARCH REPORT

| International application No. |
| --- |
| **PCT/CN2023/126403** |

| A. | CLASSIFICATION OF SUBJECT MATTER |
| --- | --- |

C12N 1/16(2006.01)i;  C12R1/645(2006.01)i

According to International Patent Classification (IPC) or to both national classification and IPC

| B. | FIELDS SEARCHED |
| --- | --- |

Minimum documentation searched (classification system followed by classification symbols)

IPC:C12N C12R

Documentation searched other than minimum documentation to the extent that such documents are included in the fields searched

Electronic data base consulted during the international search (name of data base and, where practicable, search terms used)

CJFD, DWPI; CNABS; CNTXT; CNKI; WOTXT; USTXT; EPTXT; ENTXTC; ENTXT; 万方, WANFANG: 百度学术, BAIDU SCHOLAR; BING; Pubmed; ISI web of knowledge; Elsevier Science Direct; Springerlink; NCBI; 中国专利生物序列检索系统, China Patents Biological Sequence Search System; STN: 马克斯克鲁维酵母, 克鲁维, 酵母, 乳酸, 耐乳酸, 耐酸, 生物量, M2023217, M20222110, Kluyveromyces marxianus, 安琪酵母, 孙雅芳, 覃先武, 郭天芬, 张彦, 石雨

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2017036294 A1 (FUDAN UNIVERSITY) 09 March 2017 (2017-03-09) abstract, claims 1-16, and embodiments 1-3 | 2-3, 5-16 |
| X | HENSING, M. et al. "Production of extracellular inulinase in high-cell-density fed-batch cultures of Kluyveromyces marxianus" *Appl Microbiol Biotechnol,* Vol. 42, 31 December 1994 (1994-12-31), pages 516-521, abstract, and results | 2-3, 5-16 |
| X | CN 105087403 A (FUDAN UNIVERSITY) 25 November 2015 (2015-11-25) abstract, claims 1-10, and specific embodiments | 2-3, 5-16 |
| X | CN 110951630 A (LIU JIANWEI et al.) 03 April 2020 (2020-04-03) abstract, claims 1-3, and embodiments 1-3 | 12, 14-15 |
| X | US 2014206053 A1 (BY THE SECRETARY OF AGRICULTURE THE UNITED STATESOF AMERICA, AS REPRESENTED) 24 July 2014 (2014-07-24) abstract, claims 1-9, and embodiment 1 | 12, 14-15 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **11 December 2023** | **14 December 2023** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/126403** |

**C.     DOCUMENTS CONSIDERED TO BE RELEVANT**

| Category* | Citation of document, with indication, where appropriate, of the relevant passages | Relevant to claim No. |
| --- | --- | --- |
| A | CN 115895920 A (CHINA NATIONAL RESEARCH INSTITUTE OF FOOD & FERMENTATION INDUSTRIES CO., LTD. et al.) 04 April 2023 (2023-04-04) abstract, claims 1-3, and embodiments 1-2 | 1-11, 13, 16 |

Form PCT/ISA/210 (second sheet) (July 2022)

**INTERNATIONAL SEARCH REPORT**

International application No.

**PCT/CN2023/126403**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

a.  ☑ forming part of the international application as filed.

b.  ☐ furnished subsequent to the international filing date for the purposes of international search (Rule 13*ter*.1(a)),

☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**
Information on patent family members

International application No.

**PCT/CN2023/126403**

| Patent document cited in search report | | | Publication date (day/month/year) | Patent family member(s) | | | Publication date (day/month/year) |
|---|---|---|---|---|---|---|---|
| WO | 2017036294 | A1 | 09 March 2017 | None | | | |
| CN | 105087403 | A | 25 November 2015 | None | | | |
| CN | 110951630 | A | 03 April 2020 | None | | | |
| US | 2014206053 | A1 | 24 July 2014 | WO | 2014116517 | A1 | 31 July 2014 |
| | | | | US | 8906654 | B2 | 09 December 2014 |
| CN | 115895920 | A | 04 April 2023 | None | | | |

Form PCT/ISA/210 (patent family annex) (July 2022)

**EP 4 692 307 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310540115
- CN 115287202 A
- CN 115211490 A
- CN 107287127 A
- CN 113583883 B

**Non-patent literature cited in the description**

- *List of Cultures Available in Food*, 2022

25